(19) Europäisches Patentamt
European Patent Office
Office européen des brevets

(11) Publication number : **0 536 204 B1**

(12) # EUROPEAN PATENT SPECIFICATION

(45) Date of publication of patent specification : **06.04.94 Bulletin 94/14**

(51) Int. Cl.[5] : **A61K 9/12,** A61K 9/72

(21) Application number : **91911624.4**

(22) Date of filing : **21.06.91**

(86) International application number : **PCT/US91/04423**

(87) International publication number : **WO 92/00062 09.01.92 Gazette 92/02**

(54) **THE USE OF SOLUBLE FLUOROSURFACTANTS FOR THE PREPARATION OF METERED-DOSE AEROSOL FORMULATIONS.**

(30) Priority : **27.06.90 US 544659**

(43) Date of publication of application : **14.04.93 Bulletin 93/15**

(45) Publication of the grant of the patent : **06.04.94 Bulletin 94/14**

(84) Designated Contracting States : **BE CH DE ES FR GB IT LI SE**

(56) References cited :
**US-A- 3 094 547**
**US-A- 4 352 789**
**STN International Information Services Data Base: Chemical Abstracts, Accession No.: 89(14):117545K, & JP-A-53 031 582 (DAIKIN KOGYO CO., LTD) 24 MARCH 1978**

(73) Proprietor : **MINNESOTA MINING AND MANUFACTURING COMPANY**
**3M Center, P.O. Box 33427**
**St. Paul, Minnesota 55133-3427 (US)**

(72) Inventor : **MORIS, Robert, A.**
**Post Office Box 33427**
**Saint paul, MN 55133-3427 (US)**
Inventor : **SCHULTZ, David, W.**
**Post Office Box 33427**
**Saint Paul, MN 55133-3427 (US)**
Inventor : **SCHULTZ, Robert, K.**
**Post Office Box 33427**
**Saint Paul, MN 55133-3427 (US)**
Inventor : **THIEL, Charles, G.**
**Post Office Box 33427**
**Saint Paul, MN 55133-3427 (US)**

(74) Representative : **Bowman, Paul Alan**
**LLOYD WISE, TREGEAR & CO. Norman House**
**105-109 Strand**
**London WC2R OAE (GB)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### TECHNICAL FIELD OF THE INVENTION

This invention relates to suspension aerosol formulations suitable for the administration of medicaments. More particularly, it relates to pharmaceutical suspension aerosol formulations using 1,1,1,2-tetrafluoroethane or 1,1,1,2,3,3,3-heptafluoropropane as the propellant.

### BACKGROUND OF THE INVENTION

Pharmaceutical suspension aerosol formulations currently use a mixture of liquid chlorofluorocarbons as the propellant. Fluorotrichloromethane, dichlorodifluoromethane and dichlorotetrafluoroethane are the most commonly used propellants in aerosol formulations for administration by inhalation.

Chlorofluorocarbons have been implicated in the destruction of the ozone layer and their production is being phased out. Hydrofluorocarbon 134a (HFC-134a, 1,1,1,2-tetrafluoroethane) and hydrofluorocarbon 227 (HFC-227, 1,1,1,2,3,3,3-heptafluoropropane) are viewed as being more ozone friendly than many chlorofluorocarbon propellants; furthermore, they have low toxicity and vapor pressures suitable for use in aerosols.

U.S. Pat. No. 4,352,789 discloses a self-propelling, powder dispensing aerosol composition comprising between about 0.001 and 20 percent by weight of a finely-divided solid material coated with a dry coating of a perfluorinated surface-active dispersing agent of a particular type which constitutes between 0.1 to 20 percent by weight of the coated solid and a halogenated propellant. The solid material can be a medicament. The use of 1,1,1,2-tetrafluoroethane or 1,1,1,2,3,3,3-heptafluoropropane as a propellant is not specifically disclosed.

### SUMMARY OF THE INVENTION

This invention provides suspension aerosol formulations comprising an effective amount of a powdered medicament, between 0.001 and 0.6 percent by weight of a perfluorinated surface-active dispersing agent and a propellant comprising a hydrofluorocarbon selected from the group consisting of 1,1,1,2-tetrafluoroethane and 1,1,1,2,3,3, 3-heptafluoropropane, and a mixture thereof.

The perfluorinated surface-active agent is selected from the group consisting of a perfluorinated sulfonamido alcohol phosphate ester having the general formula

$$[R_fSO_2N(R)R'O]_m\overset{O}{\overset{\|}{P}}(OH)_{3-m}$$

wherein $R_f$ is a perfluorinated radical selected from the group consisting of aliphatic $C_nF_{2n+1}$ and cycloaliphatic $C_nF_{2n-1}$, where n is an integer from 4 to 10, R is selected from the group consisting of hydrogen and alkyl having 4 to 12 carbon atoms, R' is alkylene having 2 to 8 carbon atoms and m is an integer from 1 to 3, and a mixture of two or more of said esters;

the formulation exhibiting substantially no growth in particle size or change in crystal morphology of said medicament over a prolonged period, being substantially readily redispersible, and upon redispersion not flocculating so quickly as to prevent reproducible dosing of the medicament. Preferably, the formulation is prepared by combining the dispersing agent and propellant rather than coating the dispersing agent onto the powdered medicament prior to addition of said propellant.

The pharmaceutical suspension aerosol formulations of the invention are suitable, for example, for dermal, pulmonary, or mucosal (e.g., buccal or nasal) administration.

### DETAILED DESCRIPTION OF THE INVENTION

The term "suspension aerosol" means that the medicament is in powder form and is substantially insoluble in the propellant.

By "prolonged period" as used herein in the context of crystallization is meant at least about four (4) months.

The medicament is micronized, that is, over 90 percent of the particles have a diameter of less than about 10 µm.

The medicament is generally present in an amount effective to bring about the intended therapeutic effect

of the medicament, i.e., an amount such that one or more metered volumes of the formulation contains an effective amount of the drug. The amount of medicament, however, depends on the potency of the particular medicament being formulated. Generally, the medicament constitutes from 0.01 to 5 percent by weight of the total weight of the formulation, preferably 0.01 to 2 percent by weight of the total weight of the formulation.

Medicaments for delivery by inhalation include, for example, analgesics, anginal preparations, antiallergics, antibiotics, antihistamines, antiinflammatories, antitussives, bronchodilators, enzymes, hormones, peptides, steroids, or a combination of these.

Examples of medicaments falling within the above therapeutic classes are: adrenochrome, albuterol, albuterol sulfate, atropine methylnitrate or sulfate, beclomethasone dipropionate, chlorotetracycline, codeine, colchicine, cortisone, disodium cromoglycate, ephedrine, ephedrine hydrochloride or sulfate, epinephrine bitartrate, fentanyl, flunisolide, formoterol, glucagon, heparin, hydrocortisone, hydroxytetracycline, insulin, isoproterenol hydrochloride or sulfate, morphine, nedocromide, neomycin, oscapine, penicillin, phenylephrine bitartrate or hydrochloride, phenylpropanolamine hydrochloride, pirbuterol acetate or hydrochloride, prednisolone, salmeterol, streptomycin, tetracycline, triamcinolone acetonide, and trypsin.

Preferred medicaments in the practice of this invention include albuterol, albuterol sulfate, beclomethasone dipropionate, disodium cromoglycate, epinephrine bitartrate, fenoterol hydrobromide, ipratropium bromide, isoproterenol hydrochloride, isoproterenol sulfate, metaproterenol sulfate, phenylephrine bitartrate, phenylephrine hydrochloride, pirbuterol acetate, pirbuterol hydrochloride, procaterol hydrochloride, salmeterol, triamcinolone acetonide, and mixtures thereof.

Perfluorinated surface-active dispersing agents useful in the invention are perfluorinated sulfonamido alcohol phosphate esters or mixtures of such compounds that are soluble in 1,1,1,2-tetrafluoroethane, 1,1,1,2,3,3,3-heptafluoropropane, or a mixture thereof.

Suitable perfluorinated sulfonamido alcohol phosphate esters include those described in U.S. Pat. No. 3,094,547, which is incorporated herein by reference, having the general formula:

$$[R_fSO_2N(R)R'O]_mP(=O)(OH)_{3-m}$$

where $R_f$ is a perfluorinated radical selected from the group consisting of aliphatic $C_nF_{2n+1}$ and cycloaliphatic $C_nF_{2n-1}$, where n is an integer from 4 to 10, R is selected from the group consisting of hydrogen and alkyl having 4 to 12 carbon atoms, R' is alkylene having 2 to 8 carbon atoms and m is an integer from 1 to 3.

Particularly preferred perfluorinated sulfonamido alcohol phosphate esters include bis(perfluorooctyl-N-ethylsulfonamidoethyl)phosphate, tris(perfluorooctyl-N-ethylsulfonamidoethyl)phosphate, and mixtures thereof.

For some medicaments a combination of the bis(perfluorooctyl-N-ethylsulfonamidoethyl)phosphate and the tris(perfluorooctyl-N-ethylsulfonamidoethyl)phosphate affords aerosol formulations with superior suspension qualities compared to suspensions obtained by using either ester alone. The total amount of ester and the ratio of the bis(perfluorooctyl-N-ethylsulfonamidoethyl)phosphate to the tris(perfluorooctyl-N-ethylsulfonamidoethyl)phosphate can be optimized by those skilled in the art for particular medicaments.

The perfluorinated surface-active dispersing agent preferably has a solubility of at least 0.1 percent by weight, more preferably at least 0.3 percent by weight, and most preferably at least 0.8 percent by weight in the propellant.

The perfluorinated surface-active dispersing agent constitutes from 0.001 to 0.6 percent by weight, preferably 0.001 to 0.5 percent by weight, of the aerosol formulation. The particular preferred amount depends on the particular medicament being formulated and on the particular surface-active dispersing agent being used. It is preferred to use approximately the minimum amount of agent needed to provide a suitable suspension.

The hydrofluorocarbon or mixture thereof is preferably the only propellant present in the formulations of the invention. However, one or more other propellants such as propellant 142b (1-chloro-1,1-difluoroethane) can also be present.

The suspension aerosol formulations of the invention can be prepared by first preparing a solution of the perfluorinated surface-active dispersing agent in the propellant and then suspending the medicament in the solution. In order to prepare a formulation in this manner, the perfluorinated surface-active dispersing agent is placed in an aerosol vial, a continuous valve is crimped onto the vial and the vial is pressure filled with the propellant. The vial is shaken on an automatic shaker until all of the dispersing agent is in solution. The micronized medicament is then placed in a separate aerosol vial, a continuous valve is crimped onto the vial and

the vial is pressure filled with the previously prepared solution. The medicament is then dispersed in the solution by mixing or homogenizing. If the medicament being formulated is moisture sensitive, these steps should be performed in a dehumidified atmosphere using only dry materials and equipment.

The following examples are provided to illustrate the invention but should not be construed as limiting the invention.

In the following examples the quality of the aerosol suspension is rated on a scale of 1 to 5 with 1 indicating a "poor" suspension and 5 indicating an "excellent" suspension. A poor suspension is characterized by one or more of the following: it has a rapid rate of settling or separation, it is difficult to redisperse after settling or separation, it forms large flocs quickly, or it exhibits crystal formation. In contrast, an excellent suspension is slow to settle or separate, is easily redispersed, has minimal flocculation, and exhibits no crystallization or crystal morphology changes. Substantially no crystal formation, relative ease of redispersion, and absence of rapid flocculation after redispersion are important properties in order to provide reproducible dosing of the medicament. Absence of substantial crystal formation provides for maximization of the fraction of the dose deliverable to the target area of the lung. Ease of redispersion permits dosing of a uniform suspension. Finally, rapid flocculation results in a large variation in the dose delivered from the aerosol canister. Suspensions exhibiting a rating of 1 or 2 are not considered desirable in terms of an overall balance of properties of degree of crystallization, ease of redispersibility, and nature of any flocculation, whereas ones exhibiting a rating of 3, 4 or 5 are considered desirable and fall within the scope of this invention.

As used in the Examples below, the term "diester" refers to bis(perfluorooctyl-N-ethylsulfonamidoethyl)phosphate, and the term "triester" refers to tris(perfluorooctyl-N-ethylsulfonamidoethyl)phosphate. Except as otherwise indicated the propellant in the Examples below is 1,1,1,2-tetrafluoroethane (HFC-134a).

Example 1

A 11.528 mg portion of bis(perfluorooctyl-N-ethylsulfonamidoethyl)phosphate was placed in a 4 ounce vial, the vial was sealed with a continuous valve then pressure filled with 115.65 g of 1,1,1,2-tetrafluoroethane. The vial was then shaken on an automatic shaker for 15 minutes. The resulting stock solution contained 0.01 % by weight of bis(perfluorooctyl-N-ethylsulfonamidoethyl)phosphate. A 100 mg portion of micronized albuterol sulfate was placed in a 15 cc vial along with 5 mL of glass beads, the vial was sealed with a continuous valve then pressure filled with the previously prepared stock solution. The vial was shaken on a WIG-L-BUG™ mixer for 30 seconds. The resulting suspension contained 0.5% by weight of albuterol sulfate and had a quality rating of 5 (excellent).

Examples 2-13

Using the general method of Example 1, a series of micronized albuterol sulfate suspensions were prepared. Table 1 shows the amount (percent by weight based on the total weight of the formulation) and identity of the surface-active dispersing agent (ratios are weight:weight) used and the quality rating of the suspension. The suspensions of Examples 2 and 3 contained 0.5% by weight of albuterol sulfate, that of Example 4 contained 0.46% by weight and the remaining Examples contained 0.45 % by weight of albuterol sulfate.

Table 1

| Example | Surface-Active Dispersing Agent | | Rating |
|---|---|---|---|
| 2 | 0.005% | diester | 3 |
| 3 | 0.05% | diester | 5 |
| 4 | 0.3% | diester | 3 |
| 5 | 0.005% | 3:1 diester:triester | 5 |
| 6 | 0.01% | 8:1 diester:triester | 4 |
| 7 | 0.05% | 38:1 diester:triester | 3 |
| 8 | 0.005% | 4:3 diester:triester | 5 |
| 9 | 0.01% | 8:3 diester:triester | 4 |
| 10 | 0.05% | 38:3 diester:triester | 3 |
| 11 | 0.005% | 4:13 diester:triester | 5 |
| 12 | 0.01% | 8:13 diester:triester | 5 |
| 13 | 0.05% | 38:13 diester:triester | 3 |

Examples 14-18

Using the general method of Example 1, a series of suspension aerosol formulations containing 0.5% percent by weight micronized pirbuterol hydrochloride was prepared. Table 2 shows the amount (percent by weight based on the total weight of the formulation) and identity of the surface-active dispersing agent used and the suspension quality rating.

Table 2

| Example | Surface-Active Dispersing Agent | | Rating |
|---|---|---|---|
| 14 | 0.05% | diester | 5 |
| 15 | 0.10% | diester | 5 |
| 16 | 0.15% | diester | 5 |
| 17 | 0.20% | diester | 5 |
| 18 | 0.01% | diester | 2 |

Examples 19-27

Using the general method of Example 1, a series of aerosol suspension formulations containing 1.6% by weight based on the total weight of the formulation of micronized disodium cromoglycate was prepared. Table 3 shows the amount (percent by weight based on the total weight of the formulation) and identity (ratios are weight:weight) of the surface-active dispersing agent used and the suspension quality rating.

Table 3

| Example | Surface-Active Dispersing Agent | | Rating |
|---|---|---|---|
| 19 | 0.03% | diester | 1 |
| 20 | 0.05% | diester | 1 |
| 21 | 0.01% | diester | 1 |
| 22 | 0.3% | diester | 3 |
| 23 | 0.3% | 1:1 diester:triester | 4 |
| 24 | 0.3% | triester | 3 |
| 25 | 0.05% | 1:1 diester:triester | 3 |
| 26 | 0.1% | 1:1 diester:triester | 5 |
| 27 | 0.15% | 1:1 diester:triester | 5 |

Examples 28-40

Using the general method of Example 1, a series of suspension aerosol formulations containing 0.45% by weight of micronized pirbuterol acetate was prepared. Table 4 shows the amount (percent by weight based on the total weight of the formulation) and identity (ratios are weight:weight) of the surface-active dispersing agent used and the suspension quality rating.

Table 4

| Example | Surface-Active Dispersing Agent | | Rating |
|---|---|---|---|
| 28 | 0.3% | diester | 1 |
| 29 | 0.01% | diester | 3 |
| 30 | 0.05% | diester | 2 |
| 31 | 0.10% | diester | 2 |
| 32 | 0.15% | diester | 2 |
| 33 | 0.20% | diester | 2 |
| 34 | 0.005% | 3:1 diester:triester | 2 |
| 35 | 0.005% | 4:3 diester:triester | 2 |
| 36 | 0.005% | 4:13 diester:triester | 2 |
| 37 | 0.1% | 3:1 diester:triester | 2 |
| 38 | 0.1% | 1:1 diester:triester | 2 |
| 39 | 0.3% | 3:1 diester:triester | 2 |
| 40 | 0.5% | 3:1 diester:triester | 2 |

Examples 41-46

Using the general method of Example 1, a series of suspension aerosol formulations containing 0.5% by weight based on the total weight of the formulation of micronized epinephrine bitartrate was prepared. Table 5 shows the amount (percent by weight based on the total weight of the formulation) and identity (ratios are weight:weight) of the surface-active dispersing agent used and the suspension quality rating.

Table 5

| Example | Surface-Active Dispersing Agent | | Rating |
|---|---|---|---|
| 41 | 0.05% | 1:1 diester:triester | 5 |
| 42 | 0.1% | 1:1 diester:triester | 2 |
| 43 | 0.15% | 1:1 diester:triester | 2 |
| 44 | 0.05% | diester | 4 |
| 45 | 0.1% | diester | 2 |
| 46 | 0.15% | diester | 2 |

Example 47

A 16.6 mg portion of perfluorooctyl-N-ethylsulfonamidoethylphosphate was mixed with 1 g of ethanol in a 4 gram glass vial. The resulting solution was transferred to a 4 ounce glass aerosol vial which was then sealed with a continuous valve and pressure filled with 100 g of 1,1,1,2-tetrafluoroethane to give a stock solution containing 0.016 percent by weight of the ester and 1 percent by weight of ethanol. A 100 mg portion of micronized albuterol sulfate was placed in a 15 cc glass vial along with 5 mL of glass beads, the vial was sealed with a continuous valve and then pressure filled with the stock solution. The vial was placed on a WIG-L-BUG™ mixer for at least 30 seconds. The resulting suspension contained 0.5% by weight of albuterol sulfate and had a quality rating of 2.

Example 48

Using the general method of Example 47, a suspension aerosol containing 0.5% by weight of micronized albuterol sulfate, 0.05% by weight of perfluorooctyl-N-ethylsulfonamidoethylphosphate, 1.2% by weight of ethanol and 1,1,1,2-tetrafluoroethane was prepared. The resulting suspension had a quality rating of 1.

Example 49

Using the general method of Example 47, a suspension aerosol containing 0.5% by weight of micronized albuterol sulfate, 0.005% by weight of perfluorooctyl-N-ethylsulfonamidoethylphosphate, 0.5% by weight of ethanol and 1,1,1,2-tetrafluoroethane was prepared. The resulting suspension had a quality rating of 4.

Example 50

A 10.0 mg portion of bis(perfluorooctyl-N-ethylsulfonamidoethyl)phosphate and a 50.7 mg portion of tris(perfluorooctyl-N-ethylsulfonamidoethyl)phosphate were placed in a vial, the vial was sealed with a continuous valve then pressure filled with 99.879 g of 1,1,1,2-tetrafluoroethane. The resulting stock solution contained 0.01% by weight of bis(perfluorooctyl-N-ethylsulfonamidoethyl)phosphate and 0.05% by weight of tris(perfluorooctyl-N-ethylsulfonamidoethyl)phosphate. A 30 mg portion of micronized beclomethasone dipropionate was placed in a vial along with 3 mL of glass beads, the vial was sealed with a continuous valve and pressure filled with 10 g of the previously prepared stock solution. The vial was placed on a WIG-L-BUG™ mixer for at least 30 seconds. The resulting suspension contained 0.3% by weight of beclomethasone dipropionate and had a quality suspension rating of 4 (excellent).

Examples 51-55

Using the general method of Example 50 and the stock solution prepared in Example 50, a series of suspension aerosols was prepared. Table 6 shows the amount (percent by weight based on the total weight of the formulation) and identity of the medicament used and the quality rating of the suspension. All of the suspensions contained 0.01% by weight of bis(perfluorooctyl-N-ethylsulfonamidoethyl)phosphate and 0.05% by weight of tris(perfluorooctyl-N-ethylsulfonamidoethyl)phosphate.

Table 6

| Example | Medicament | | Rating |
|---|---|---|---|
| 51 | 0.3% | triamcinolone acetonide | 5 |
| 52 | 0.5% | pirbuterol acetate | 5 |
| 53 | 1.5% | disodium cromoglycate | 5 |
| 54 | 0.5% | albuterol sulfate | 5 |
| 55 | 0.45% | salmeterol | 3 |

Examples 56-58

Using the general method of Example 1, a series of suspension aerosol formulations containing 0.1% by weight of micronized salmeterol was prepared. Table 7 shows the amount (percent by weight based on the total weight of the formulation) and identity of the surface-active dispersing agent used and the suspension quality rating.

Table 7

| Example | Surface-Active Dispersing Agent | | Rating |
|---|---|---|---|
| 56 | 0.01% | diester | 4 |
| 57 | 0.005% | diester | 5 |
| 58 | 0.001% | diester | 5 |

Examples 59-64

A series of suspension aerosol formulations in which 1,1,1,2,3,3,3-heptafluoropropane (HFC-227) serves as the propellant was prepared using the general method of Example 1. Table 8 shows the amount (percent by weight based on the total weight of the formulation) and identity of the surface-active dispersing agent used and the suspension quality rating. The formulations of Examples 59-61 contained 0.3 percent by weight based on the total weight of the formulation of micronized triamcinolone acetonide. Those of Examples 62-64 contained 0.5 percent by weight of micronized pirbuterol acetate.

Table 8

| Example | Surface-Active Dispersing Agent | | Rating |
|---|---|---|---|
| 59 | 0.025% | diester | 4 |
| 60 | 0.05% | 1:4 diester:triester | 5 |
| 61 | 0.005% | 4:1 diester:triester | 5 |
| 62 | 0.025% | diester | 5 |
| 63 | 0.05% | 1:4 diester:triester | 4 |
| 64 | 0.005% | 4:1 diester:triester | 4 |

In the claims that follow, all weight percentages are based on the total weight of the formulation unless otherwise stated.

## Claims

1. A suspension aerosol formulation, comprising: a propellant comprising a hydrofluorocarbon selected from the group consisting of 1,1,1,2-tetrafluoroethane and 1,1,1,2,3,3,3-heptafluoropropane, and a mixture thereof; a therapeutically effective amount of a powdered medicament; and between 0.001 and 0.6 percent by weight of a surface-active dispersing agent of the formula

$$[R_fSO_2N(R)R'O]_m\overset{O}{\overset{\|}{P}}(OH)_{3-m}$$

wherein $R_f$ is a perfluorinated radical selected from the group consisting of aliphatic $C_nF_{2n+1}$ and cycloaliphatic $C_nF_{2n-1}$ where n is an integer from 4 to 10, R is selected from the group consisting of hydrogen and alkyl having 4 to 12 carbon atoms, R' is alkylene having 2 to 8 carbon atoms and m is an integer from 1 to 3, and mixture of two or more of said esters;
the formulation exhibiting substantially no growth in particle size or change in crystal morphology of said medicament over a prolonged period, being substantially readily redispersible, and upon redispersion not flocculating so quickly as to prevent reproducible dosing of the medicament.

2. A suspension aerosol formulation according to Claim 1 wherein said powdered medicament is present in an amount of 0.01 to 2 percent by weight; said formulation being prepared by combining said dispersing agent and propellant rather than coating said dispersing agent onto said powdered medicament prior to addition of said propellant.

3. A suspension aerosol formulation according to Claim 1 wherein said dispersing agent is present in an amount of 0.001 to 0.5 percent by weight.

4. A suspension aerosol formulation according to Claim 1 wherein said dispersing agent has a solubility of at least 0.3 percent by weight in said propellant.

5. A suspension aerosol formulation according to Claim 4 wherein said dispersing agent has a solubility of at least 0.8 percent by weight in said propellant.

6. A suspension aerosol formulation according to Claim 1 wherein said dispersing agent is selected from the group consisting of bis(perfluorooctyl-N-ethylsulfonamidoethyl)phosphate, tris(perfluorooctyl-N-ethylsulfonamidoethyl)phosphate, and mixtures thereof.

7. A suspension aerosol formulation according to Claim 1 wherein said medicament is selected from the group consisting of an analgesic, an anginal preparation, an antiallergic, an antibiotic, an antihistamine, an antiinflammatory, an antitussive, a bronchodilator, an enzyme, a hormone, a peptide, a steroid, and mixtures thereof.

8. A suspension aerosol formulation according to Claim 1 wherein said medicament is selected from the group consisting of albuterol, albuterol sulfate, beclomethasone dipropionate, disodium cromoglycate, epinephrine bitartrate, fenoterol hydrobromide, ipratropium bromide, isoproterenol hydrochloride, isoproterenol sulfate, metaproterenol sulfate, phenylephrine bitartrate, phenylephrine hydrochloride, pirbuterol acetate, pirbuterol hydrochloride, procaterol hydrochloride, salmeterol, triamcinolone acetonide, and mixtures thereof.

9. A suspension aerosol formulation according to Claim 1 wherein 1,1,1,2-tetrafluoroethane is essentially the only propellant, and comprising between 0.1 and 1.0 percent by weight of albuterol sulfate having a substantially uniform particle size of less than about 10 μm in diameter, and between 0.008 and 0.06 percent by weight of bis(perfluorooctyl-N-ethylsulfonamidoethyl)phosphate.

10. A suspension aerosol formulation according to Claim 1 wherein 1,1,1,2-tetrafluoroethane is essentially the only propellant, and comprising between 0.5 and 2 percent by weight of disodium cromoglycate having a substantially uniform particle size of less than 10 μm in diameter, and between 0.05 and 0.4 percent by weight of a mixture of bis(perfluorooctyl-N-ethylsulfonamidoethyl)phosphate and tris(perfluorooctyl-N-ethylsulfonamidoethyl)phosphate.

11. A suspension aerosol formulation according to Claim 10 wherein said bis(perfluorooctyl-N-ethylsulfonamidoethyl)phosphate and said tris(perfluorooctyl-N-ethylsulfonamidoethyl)phosphate are present in about equal amounts by weight.

12. A suspension aerosol formulation according to Claim 1 wherein 1,1,1,2-tetrafluoroethane is essentially the only propellant, and comprising between 0.1 and 1 percent by weight of epinephrine bitartrate having a substantially uniform particle size of less than about 10 microns in diameter, and between 0.02 and 0.07 percent by weight of a mixture of bis(perfluorooctyl-N-ethylsulfonamidoethyl)phosphate and tris(perfluorooctyl-N-ethylsulfonamidoethyl)phosphate.

13. A suspension aerosol formulation according to Claim 12 wherein said bis(perfluorooctyl-N-ethylsulfonamidoethyl)phosphate and said tris(perfluorooctyl-N-ethylsulfonamidoethyl)phosphate are present in about equal amounts by weight.

14. A suspension aerosol formulation according to Claim 1 wherein 1,1,1,2-tetrafluoroethane is essentially the only propellant, and comprising between 0.1 and 1 percent by weight of epinephrine bitartrate having a substantially uniform particle size of less than 10 μm in diameter, and between about 0.02 and about 0.07 percent by weight of bis(perfluorooctyl-N-ethylsulfonamidoethyl)phosphate.

15. A suspension aerosol formulation according to Claim 1 wherein 1,1,1,2-tetrafluoroethane is essentially the only propellant, and comprising between 0.1 and 1 percent by weight of pirbuterol hydrochloride having a substantially uniform particle size of less than about 10 microns in diameter, and between 0.03 and 0.3 percent by weight of bis(perfluorooctyl-N-ethylsulfonamidoethyl)phosphate.

16. A suspension aerosol formulation according to Claim 1 comprising between about 0.1 and about 1.0 percent by weight of albuterol sulfate having a substantially uniform particle size of less than 10 μm in diameter, and between 0.004 and 0.02 percent by weight of a mixture of bis(perfluorooctyl-N-ethylsulfonamidoethyl)phosphate and tris(perfluorooctyl-N-ethylsulfonamidoethyl)phosphate, with the proviso that the ratio by weight of said bis ester to said tris ester is 8:1 to 1:4.

17. A suspension aerosol formulation according to Claim 1, prepared by combining the dispersing agent and the propellant rather than coating the dispersing agent onto the powdered medicament prior to addition of said propellant.

18. A suspension aerosol formulation according to Claim 1 comprising 1,1,1,2-tetrafluoroethane as essentially the only propellant.

19. A suspension aerosol formulation according to Claim 1 comprising 1,1,1,2,3,3,3-heptafluoropropane as essentially the only propellant.

20. A suspension aerosol formulation according to Claim 1 wherein 1,1,1,2-tetrafluoroethane is essentially the only propellant, and comprising: 0.02 to 0.07 percent by weight of a mixture of about one part by weight bis(perfluorooctyl-N-ethyl sulfonamidoethyl)phosphate and about five parts by weight tris(perfluorooctyl-N-ethylsulfonamidoethyl)phosphate; and a medicament having substantially uniform particle size of less than 10 μm in diameter selected from the group consisting of beclomethasone dipropionate in an amount of 0.1 to 0.5 percent by weight, triamcinolone acetonide in an amount of 0.1 to 0.5 percent by weight, pirbuterol acetate in an amount of 0.3 to 0.7 percent by weight, disodium chromoglycate in an amount of 1.0 to 2.0 percent by weight, albuterol sulfate in an amount of 0.3 to 0.7 percent by weight, and salmeterol in an amount of 0.4 to 0.5 percent by weight.

21. A suspension aerosol formulation according to Claim 1 wherein 1,1,1,2-tetrafluoroethane is essentially the only propellant, and comprising 0.05 to 0.2 percent by weight of salmeterol having a substantially uniform particle size of less than 10 μm in diameter and 0.001 to 0.01 percent by weight bis(perfluorooctyl-N-ethylsulfonamidoethyl)phosphate.

22. A suspension aerosol formulation according to Claim 1 wherein 1,1,1,2,3,3,3-heptafluoropropane is essentially the only propellant and comprising 0.1 to 0.5 percent by weight triamcinolone acetonide having a substantially uniform particle size of less than 10 µm in diameter and 0.005. to 0.05 percent by weight of a dispersing agent selected from the group consisting of bis(perfluorooctyl-N-ethylsulfonamidoethyl)phosphate and a mixture of bis(perfluorooctyl-N-ethylsulfonamidoethyl)phosphate and tris(perfluorooctyl-N-ethylsulfonamidoethyl)phosphate.

23. A suspension aerosol formulation according to Claim 1 wherein 1,1,1,2,3,3,3-heptafluoropropane is essentially the only propellant and comprising 0.3 to 0.7 percent by weight pirbuterol acetate having a substantially uniform particle size of less than 10 µm in diameter and 0.005 to 0.05 percent by weight of a dispersing agent selected from the group consisting of bis(perfluorooctyl-N-ethylsulfonamidoethyl)phosphate and a mixture of bis(perfluorooctyl-N-ethylsulfonamidoethyl)phosphate and tris(perfluorooctyl-N-ethylsulfonamidoethyl)phosphate.

## Patentansprüche

1. Aerosolsuspensionsformulierung, die umfaßt: ein einen Fluorkohlenwasserstoff umfassendes Treibmittel, ausgewählt aus 1,1,1,2-Tetrafluorethan und 1,1,1,2,3,3,3-Heptafluorpropan und einem Gemisch davon, eine therapeutisch wirksame Menge eines pulverisierten Arzneimittels und zwischen 0,001 und 0,6 Gewichtsprozent oberflächenaktives Dispergiermittel der Formel

$$[R_fSO_2N(R)R'O]_m\overset{O}{\overset{\|}{P}}(OH)_{3-m}$$

in der $R_f$ einen perfluorierten Rest bedeutet, der aus aliphatischen $C_nF_{2n+1}$- und cycloaliphatischen $C_nF_{2n-1}$-Resten ausgewählt ist, wobei n eine ganze Zahl von 4 bis 10 ist, R aus einem Wasserstoffatom und einem Alkylrest mit 4 bis 12 Kohlenstoffatomen ausgewählt ist, R' einen Alkylenrest mit 2 bis 8 Kohlenstoffatomen darstellt und m eine ganze Zahl von 1 bis 3 ist, und ein Gemisch aus zwei oder mehreren der Ester;

wobei die Formulierung im wesentlichen kein Wachstum in der Teilchengröße oder keine Änderung in der Kristallmorphologie des Arzneimittels über einen längeren Zeitraum zeigt und im wesentlichen leicht wiederholt dispergierbar ist und nach erneuter Dispersion nicht so schnell ausflockt, daß eine reproduzierbare Dosierung des Arzneimittels verhindert wird.

2. Aerosolsuspensionsformulierung nach Anspruch 1, wobei das pulverisierte Arzneimittel in einer Menge von 0,01 bis 2 Gewichtsprozent vorliegt, wobei die Formulierung durch Kombinieren des Dispergiermittels mit dem Treibgas anstatt durch Beschichten des pulverisierten Arzneimittels mit dem Dispergiermittel vor Zusatz des Treibgases hergestellt wird.

3. Aerosolsuspensionsformulierung nach Anspruch 1, wobei das Dispergiermittel in einer Menge von 0,001 bis 0,5 Gewichtsprozent vorliegt.

4. Aerosolsuspensionsformulierung nach Anspruch 1, wobei das Dispergiermittel eine Löslichkeit von mindestens 0,3 Gewichtsprozent in dem Treibgas aufweist.

5. Aerosolsuspensionsformulierung nach Anspruch 4, wobei das Dispergiermittel eine Löslichkeit von mindestens 0,8 Gewichtsprozent in dem Treibgas aufweist.

6. Aerosolsuspensionsformulierung nach Anspruch 1, wobei das Dispergiermittel aus Bis(perfluoroctyl-N-ethylsulfonamidoethyl)phosphat, Tris(perfluoroctyl-N-ethylsulfonamidoethyl)phosphat und Gemischen davon ausgewählt ist.

7. Aerosolsuspensionsformulierung nach Anspruch 1, wobei das Arzneimittel aus einem Analgetikum, einem Anginapräparat, einem Antiallergikum, einem Antibiotikum, einem Antihistaminikum, einem entzündungshemmenden Mittel, einem Hustenmittel, einem Bronchodilatator, einem Enzym, einem Hormon, einem Peptid, einem Steroid und Gemischen davon ausgewählt ist.

8. Aeresolsuspensionsformulierung nach Anspruch 1, wobei das Arzneimittel aus Albuterol, Albuterolsulfat, Beclomethasondipropionat, Dinatriumcromoglykat, Epinephrinbitartrat, Fenoterolhydrobromid, Ipratropiumbromid, Isoproterenolhydrochlorid, Isoproterenolsulfat, Metaproterenolsulfat, Phenylephrinbitartrat, Phenylephrinhydrochlorid, Pirbuterolacetat, Pirbuterolhydrochlorid, Procaterolhydrochlorid, Salmeterol, Triamcinolonacetonid und Gemischen davon ausgewählt ist.

9. Aerosolsuspensionsformulierung nach Anspruch 1, wobei 1,1,1,2-Tetrafluorethan im wesentlichen das einzige Treibgas ist und die zwischen 0,1 und 1,0 Gewichtsprozent Albuterolsulfat mit einer im wesentlichen gleichmäßigen Teilchengröße von weniger als etwa 10 μm im Durchmesser und zwischen 0,008 und 0,06 Gewichtsprozent Bis(perfluoroctyl-N-ethylsulfonamidoethyl)phosphat umfaßt.

10. Aerosolsuspensionsformulierung nach Anspruch 1, wobei 1,1,1,2-Tetrafluorethan im wesentlichen das einzige Treibgas ist und die zwischen 0,5 und 2 Gewichtsprozent Dinatriumcromoglykat mit einer im wesentlichen gleichmäßigen Teilchengröße von weniger als 10 μm im Durchmesser und zwischen 0,05 und 0,4 Gewichtsprozent eines Gemisches aus Bis(perfluoroctyl-N-ethylsulfonamidoethyl)phosphat und Tris(perfluoroctyl-N-ethylsulfonamidoethyl)phosphat umfaßt.

11. Aerosolsuspensionsformulierung nach Anspruch 10, wobei das Bis(perfluoroctyl-N-ethylsulfonamidoethyl)phosphat und das Tris(perfluoroctyl-N-ethylsulfonamidoethyl)phosphat in etwa gleichen Gewichtsmengen vorliegen.

12. Aerosolsuspensionsformulierung nach Anspruch 1, wobei 1,1,1,2-Tetrafluorethan im wesentlichen das einzige Treibgas ist und die zwischen 0,1 und 1 Gewichtsprozent Epinephrinbitartrat mit einer im wesentlichen gleichmäßigen Teilchengröße von weniger als etwa 10 Mikrometern im Durchmesser und zwischen 0,02 und 0,07 Gewichtsprozent eines Gemisches aus Bis(perfluoroctyl-N-ethylsulfonamidoethyl)phosphat und Tris(perfluoroctyl-N-ethylsulfonamidoethyl)phosphat umfaßt.

13. Aerosolsuspensionsformulierung nach Anspruch 12, wobei das Bis(perfluoroctyl-N-ethylsulfonamidoethyl)phosphat und das Tris(perfluoroctyl-N-ethylsulfonamidoethyl)phosphat in etwa gleichen Gewichtsmengen vorliegen.

14. Aerosolsuspensionsformulierung nach Anspruch 1, wobei 1,1,1,2-Tetrafluorethan im wesentlichen das einzige Treibgas ist und die zwischen 0,1 und 1 Gewichtsprozent Epinephrinbitartrat mit einer im wesentlichen gleichmäßigen Teilchengröße von weniger als 10 μm im Durchmesser und zwischen etwa 0,02 und etwa 0,07 Gewichtsprozent Bis(perfluoroctyl-N-ethylsulfon amidoethyl)phosphat umfaßt.

15. Aerosolsuspensionsformulierung nach Anspruch 1, wobei 1,1,1,2-Tetrafluorethan im wesentlichen das einzige Treibgas ist und die zwischen 0,1 und 1 Gewichtsprozent Pirbuterolhydrochlorid mit einer im wesentlichen gleichmäßigen Teilchengröße von weniger als etwa 10 Mikrometern im Durchmesser und zwischen 0,03 und 0,3 Gewichtsprozent Bis(perfluoroctyl-N-ethylsulfonamidoethyl)phosphat umfaßt.

16. Aerosolsuspensionsformulierung nach Anspruch 1, die zwischen etwa 0,1 und etwa 1,0 Gewichtsprozent Albuterolsulfat mit einer im wesentlichen gleichmäßigen Teilchengröße von weniger als 10 μm im Durchmesser und zwischen 0,004 und 0,02 Gewichtsprozent eines Gemisches aus Bis(perfluoroctyl-N-ethylsulfonamidoethyl)phosphat und Tris(perfluoroctyl-N-ethylsulfonamidoethyl)phosphat umfaßt, mit der Maßgabe, daß das Gewichtsverhältnis vom Bisester zum Trisester 8:1 bis 1:4 beträgt.

17. Aerosolsuspensionsformulierung nach Anspruch 1, die durch Kombinieren des Dispergiermittels mit dem Treibgas anstatt durch Beschichten des pulverisierten Arzneimittels mit dem Dispergiermittel vor Zusatz des Treibgases hergestellt wird.

18. Aerosolsuspensionsformulierung nach Anspruch 1, die 1,1,1,2-Tetrafluorethan als im wesentlichen einziges Treibgas umfaßt.

19. Aerosolsuspensionsformulierung nach Anspruch 1, die 1,1,1,2,3,3,3-Heptafluorpropan als im wesentlichen einziges Treibgas umfaßt.

20. Aerosolsuspensionsformulierung nach Anspruch 1, wobei 1,1,1,2-Tetrafluorethan im wesentlichen das einzige Treibgas ist und die umfaßt: 0,02 bis 0,07 Gewichtsprozent eines Gemisches aus etwa einem Gewichtsteil Bis(perfluoroctyl-N-ethylsulfonamidoethyl)phosphat und etwa fünf Gewichtsteilen

Tris(perfluoroctyl-N-ethylsulfonamidoethyl)phosphat und ein Arzneimittel mit im wesentlichen gleichmäßiger Teilchengröße von weniger als 10 µm im Durchmesser, das aus Beclomethasondipropionat in einer Menge von 0,1 bis 0,5 Gewichtsprozent, Triamcinolonacetonid in einer Menge von 0,1 bis 0,5 Gewichtsprozent, Pirbuterolacetat in einer Menge von 0,3 bis 0,7 Gewichtsprozent, Dinatriumcromoglykat in einer Menge von 1,0 bis 2,0 Gewichtsprozent, Albuterolsulfat in einer Menge von 0,3 bis 0,7 Gewichtsprozent und Salmeterol in einer Menge von 0,4 bis 0,5 Gewichtsprozent ausgewählt ist.

**21.** Aerosolsuspensionsformulierung nach Anspruch 1, wobei 1,1,1,2-Tetrafluorethan im wesentlichen das einzige Treibgas ist und die 0,05 bis 0,2 Gewichtsprozent Salmeterol mit einer im wesentlichen gleichmäßigen Teilchengröße von weniger als 10 µm im Durchmesser und 0,001 bis 0,01 Gewichtsprozent Bis-(perfluoroctyl-N-ethylsulfonamidoethyl)phosphat umfaßt.

**22.** Aerosolsuspensionsformulierung nach Anspruch 1, wobei 1,1,1,2,3,3,3-Heptafluorpropan im wesentlichen das einzige Treibgas ist und die 0,1 bis 0,5 Gewichtsprozent Triamcinolonacetonid mit einer im wesentlichen gleichmäßigen Teilchengröße von weniger als 10 µm im Durchmesser und 0,005 bis 0,05 Gewichtsprozent eines Dispergiermittels umfaßt, das aus Bis(perfluoroctyl-N-ethylsulfonamidoethyl)phosphat und einem Gemisch aus Bis(perfluoroctyl-N-ethylsulfonamidoethyl)phosphat und Tris(perfluoroctyl-N-ethylsulfonamidoethyl)phosphat ausgewählt ist.

**23.** Aerosolsuspensionsformulierung nach Anspruch 1, wobei 1,1,1,2,3,3,3-Heptafluorpropan im wesentlichen das einzige Treibgas ist und die 0,3 bis 0,7 Gewichtsprozent Pirbuterolacetat mit einer im wesentlichen gleichmäßigen Teilchengröße von weniger als 10 µm im Durchmesser und 0,005 bis 0,05 Gewichtsprozent eines Dispergiermittels umfaßt, das aus Bis(perfluoroctyl-N-ethylsulfonamidoethyl)phosphat und einem Gemisch aus Bis(perfluoroctyl-N-ethylsulfonamidoethyl)phosphat und Tris(perfluoroctyl-N-ethylsulfonamidoethyl)phosphat ausgewählt ist.

## Revendications

**1.** Formulation pour aérosol en suspension comprenant un agent de propulsion comprenant un hydrofluorocarbone choisi dans le groupe comprenant le 1,1,1,2-tétrafluoroéthane, le 1,1,1,2,3,3,3-heptafluoropropane et leurs mélanges, une quantité thérapeutiquement efficace d'un médicament pulvérulent et entre 0,001 et 0,6 % en poids d'un agent dispersant tensioactif de la formule :

$$[R_fSO_2N(R)R'O]_m\overset{O}{\overset{\|}{P}}(OH)_{3-m}$$

dans laquelle $R_f$ est un radical perfluoré choisi dans le groupe comprenant les radicaux aliphatiques $C_nF_{2n+1}$ et cycloaliphatiques $C_nF_{2n-1}$, où n est un nombre entier de 4 à 10, R est choisi dans le groupe comprenant l'hydrogène et les groupes alkyle comportant 4 à 12 atomes de carbone, R' est un groupe alkylène comportant 2 à 8 atomes de carbone et m est un nombre entier de 1 à 3, ou d'un mélange de deux ou plusieurs de ces esters, la formulation ne montrant pratiquement aucune croissance de la taille des particules ou modification de la morphologie cristalline du médicament susdit sur une période de temps prolongée, étant sensiblement facilement redispersable et lors d'une redispersion ne floculant pas d'une manière si rapide au point d'empêcher tout dosage reproductible du médicament.

**2.** Formulation pour aérosol en suspension suivant la revendication 1, dans laquelle le médicament pulvérulent est présent en une quantité de 0,01 à 2 % en poids, ladite formulation étant préparée en combinant l'agent dispersant et l'agent de propulsion plutôt qu'en appliquant l'agent dispersant sur le médicament pulvérulent avant l'addition de l'agent de propulsion.

**3.** Formulation pour aérosol en suspension suivant la revendication 1, dans laquelle l'agent dispersant est présent en une quantité de 0,001 à 0,5 % en poids.

**4.** Formulation pour aérosol en suspension suivant la revendication 1, dans laquelle l'agent dispersant a une solubilité d'au moins 0,3 % en poids dans l'agent de propulsion.

5. Formulation pour aérosol en suspension suivant la revendication 4, dans laquelle l'agent dispersant a une solubilité d'au moins 0,8 % en poids dans l'agent de propulsion.

6. Formulation pour aérosol en suspension suivant la revendication 1, dans laquelle l'agent dispersant est choisi dans le groupe comprenant le bis(perfluorooctyl-N-éthylsulfonamidoéthyl)phosphate, le tris(perfluorooctyl-N-éthylsulfonamidoéthyl)phosphate et leurs mélanges.

7. Formulation pour aérosol en suspension suivant la revendication 1, dans laquelle le médicament est choisi dans le groupe comprenant un analgésique, une préparation angineuse, un antiallergique, un antibiotique, une antihistamine, un antiinflammatoire, un antitoux, un bronchodilatateur, une enzyme, une hormone, un peptide, un stéroïde et leurs mélanges.

8. Formulation pour aérosol en suspension suivant la revendication 1, dans laquelle le médicament est choisi dans le groupe comprenant l'albutérol, le sulfate d'albutérol, le dipropionate de béclométhasone, le cromoglycate disodique, le bitartrate d'épinéphrine, le bromhydrate de fénotérol, le bromure d'ipratropium, le chlorhydrate d'isoprotérénol, le sulfate d'isoprotérénol, le sulfate de métaprotérénol, le bitartrate de phényléphrine, le chlorhydrate de phényléphrine, l'acétate de pirbutérol, le chlorhydrate de pirbutérol, le chlorhydrate de procatérol, le salmétérol, l'acétonide de triamcinolone et leurs mélanges.

9. Formulation pour aérosol en suspension suivant la revendication 1, dans laquelle le 1,1,1,2-tétrafluoroéthane est essentiellement le seul agent de propulsion, et comprenant entre 0,1 et 1,0 % en poids de sulfate d'albutérol ayant une taille de particule sensiblement uniforme de moins de 10 μm de diamètre et entre 0,008 et 0,06 % en poids de bis(perfluorooctyl-N-éthylsulfonamidoéthyl)phosphate.

10. Formulation pour aérosol en suspension suivant la revendication 1, dans laquelle le 1,1,1,2-tétrafluoroéthane est essentiellement le seul agent de propulsion, et comprenant entre 0,5 et 2 % en poids de cromoglycate disodique ayant une taille de particule sensiblement uniforme de moins de 10 μm de diamètre et entre 0,05 et 0,4 % en poids d'un mélange de bis(perfluorooctyl-N-éthylsulfonamidoéthyl)phosphate et de tris(perfluorooctyl-N-éthylsulfonamidoéthyl)phosphate.

11. Formulation pour aérosol en suspension suivant la revendication 10, dans laquelle le bis(perfluorooctyl-N-éthylsulfonamidoéthyl)phosphate et le tris(perfluorooctyl-N-éthylsulfonamidoéthyl)phosphate sont présents en des quantités en poids environ égales.

12. Formulation pour aérosol en suspension suivant la revendication 1, dans laquelle le 1,1,1,2-tétrafluoroéthane est essentiellement le seul agent de propulsion, et comprenant entre 0,1 et 1 % en poids de bitartrate d'épinéphrine ayant une taille de particule sensiblement uniforme de moins de 10 μm de diamètre et entre 0,02 et 0,07 % en poids d'un mélange de bis(perfluorooctyl-N-éthylsulfonamidoéthyl)phosphate et de tris(perfluorooctyl-N-éthylsulfonamidoéthyl)phosphate.

13. Formulation pour aérosol en suspension suivant la revendication 12, dans laquelle le bis(perfluorooctyl-N-éthylsulfonamidoéthyl)phosphate et le tris(perfluorooctyl-N-éthylsulfonamidoéthyl)phosphate sont présents en des quantités en poids environ égales.

14. Formulation pour aérosol en suspension suivant la revendication 1, dans laquelle le 1,1,1,2-tétrafluoroéthane est essentiellement le seul agent de propulsion, et comprenant entre 0,1 et 1 % en poids de bitartrate d'épinéphrine ayant une taille de particule sensiblement uniforme de moins de 10 μm de diamètre et entre 0,02 et 0,07 % en poids de bis(perfluorooctyl-N-éthylsulfonamidoéthyl)phosphate.

15. Formulation pour aérosol en suspension suivant la revendication 1, dans laquelle le 1,1,1,2-tétrafluoroéthane est essentiellement le seul agent de propulsion, et comprenant entre 0,1 et 1 % en poids de chlorhydrate de pirbutérol ayant une taille de particule sensiblement uniforme de moins de 10 μm de diamètre et entre 0,03 et 0,3 % en poids de bis(perfluorooctyl-N-éthylsulfonamidoéthyl)phosphate.

16. Formulation pour aérosol en suspension suivant la revendication 1, comprenant entre 0,1 et 1,0 % en poids de sulfate d'albutérol ayant une taille de particule sensiblement uniforme de moins de 10 μm de diamètre et entre 0,004 et 0,02 % en poids d'un mélange de bis (perfluorooctyl-N-éthylsulfonamidoéthyl)phosphate et de tris(perfluorooctyl-N-éthylsulfonamidoéthyl)phosphate, à la condition que le rapport en poids de l'ester bis à l'ester tris soit de 8/1 à 1/4.

17. Formulation pour aérosol en suspension suivant la revendication 1, préparée en combinant l'agent dispersant et l'agent de propulsion plutôt qu'en appliquant l'agent dispersant sur le médicament pulvérulent avant l'addition de l'agent de propulsion.

18. Formulation pour aérosol en suspension suivant la revendication 1, comprenant du 1,1,1,2-tétrafluoroéthane comme essentiellement le seul agent de propulsion.

19. Formulation pour aérosol en suspension suivant la revendication 1, comprenant du 1,1,1,2,3,3,3-heptafluoropropane comme essentiellement le seul agent de propulsion.

20. Formulation pour aérosol en suspension suivant la revendication 1, dans laquelle le 1,1,1,2-tétrafluoroéthane est essentiellement le seul agent de propulsion, et comprenant 0,02 à 0,07 % en poids d'un mélange d'une partie en poids de bis(perfluorooctyl-N-éthylsuflonamidoéthyl)phosphate et de 5 parties en poids de tris(perfluorooctyl-N-éthylsuflonamidoéthyl)phosphate et un médicament ayant une taille de particule sensiblement uniforme de moins de 10 µm de diamètre choisi dans le groupe comprenant le dipropionate de béclométhasone en une quantité de 0,1 à 0,5 % en poids, l'acétonide de triamcinolone en une quantité de 0,1 à 0,5 % en poids, l'acétate de pirbutérol en une quantité de 0,3 à 0,7 % en poids, le cromoglycate disodique en une quantité de 1,0 à 2,0 % en poids, le sulfate d'albutérol en une quantité de 0,3 à 0,7 % en poids et le salmétérol en une quantité de 0,4 à 0,5 % en poids.

21. Formulation pour aérosol en suspension suivant la revendication 1, dans laquelle le 1,1,1,2-tétrafluoroéthane est essentiellement le seul agent de propulsion, et comprenant 0,05 à 0,2 % en poids de salmétérol ayant une taille de particule sensiblement uniforme de moins de 10 µm de diamètre et 0,001 à 0,01 % en poids de bis(perfluorooctyl-N-éthylsulfonamidoéthyl)phosphate.

22. Formulation pour aérosol en suspension suivant la revendication 1, dans laquelle le 1,1,1,2,3,3,3-heptafluoropropane est essentiellement le seul agent de propulsion, et comprenant 0,1 à 0,5 % en poids d'acétonide de triamcinolone ayant une taille de particule sensiblement uniforme de moins de 10 µm de diamètre et 0,005 à 0,05 % en poids d'un agent dispersant choisi dans le groupe comprenant le bis(perfluorooctyl-N-éthylsulfonamidoéthyl)phosphate et les mélanges de bis(perfluorooctyl-N-éthylsulfonamidoéthyl)phosphate et de tris(perfluorooctyl-N-éthylsulfonamidoéthyl)phosphate.

23. Formulation pour aérosol en suspension suivant la revendication 1, dans laquelle le 1,1,1,2,3,3,3-heptafluoropropane est essentiellement le seul agent de propulsion, et comprenant 0,3 à 0,7 % en poids d'acétate de pirbutérol ayant une taille de particule sensiblement uniforme de moins de 10 µm de diamètre et 0,005 à 0,05 % en poids d'un agent dispersant choisi dans le groupe comprenant le bis(perfluorooctyl-N-éthylsulfonamidoéthyl)phosphate et les mélanges de bis(perfluorooctyl-N-éthylsulfonamidoéthyl) phosphate et de tris(perfluorooctyl-N-éthylsulfonamidoéthyl)phosphate.